# EUROPEAN PATENT APPLICATION

(11) **EP 4 835 539 A1**
(43) Date of publication of application: **04.11.2026**
(21) Application number: 25172944.8
(22) Date of filing: 28.04.2025
(51) Int. Cl.: G02B 21/00, A61B 90/20, G02B 21/20, G02B 21/36

(54) **SURGICAL OR SCIENTIFIC IMAGING DEVICE, AUXILIARY OPTICAL SYSTEM AND SYSTEM**

(71) Applicant: Leica Instruments (Singapore) Pte Ltd., Singapore 618299 (SG)
(72) Inventor: JUNG, David, 618299 Singapore (SG); AYYILDIZ, Erdem, 618299 Singapore (SG)
(74) Representative: 2SPL Patentanwälte PartG mbB

(57) **Abstract**

Examples relate to a surgical or scientific imaging device, an auxiliary optical system, and a system comprising both. A surgical or scientific imaging device (100b) comprises a plurality of optical components (120, 130, 140) arranged within the optical path of the device, at least one optical imaging sensor (150) configured to generate image data based on light transmitted via the optical path during a digital operation of the device, and an interface (160b) for attaching an auxiliary optical system (300) to the surgical or scientific imaging device in case of unavailability of the digital operation. The interface provides access to the surgical or scientific imaging device for inserting an optical component (315) of the auxiliary optical system into the optical path of the device, thereby diverting light transmitted via the optical path to the auxiliary optical system.

## Description

### Technical field

Examples relate to a surgical or scientific imaging device, an auxiliary optical system, and a system comprising a surgical or scientific imaging device and an auxiliary optical system.

### Background

Conventional surgical microscopes have a visual optical path that provides vision to the user via oculars. Some surgical microscopes additionally feature a digital imaging path using cameras. In the event of a system or power supply failure (or another problem causing the system to crash), the digital imaging path might not provide an image, but the surgical procedure can be continued using the visual optical path despite the lack of power or problem. However, the image provided by the visual optical path might be compromised due to a lack of illumination.

Fully digital microscopes rely on the power supply to show an image. A purely digital microscope may be unable to provide an image in such a failure, so that the surgery is aborted in case of power failure. Some fully digital microscopes are delivered with magnifying glasses as a backup should the microscope system fail. However, such magnifying glasses provide much lower performance than the original microscope.

There may be a desire to improve the operation of a fully digital microscope, e.g., in case of power loss.

### Summary

This desire is addressed by the subject matter of the independent claims.

Various examples of the present disclosure are based on the finding that, in case of power loss during operation of a fully digital surgical or scientific imaging device, it is primarily the digital imaging pipeline-comprising image acquisition, processing of the acquired images, and outputting of the acquired images-that is impacted by the failure, while the optical components of the surgical or scientific imaging device are still operational. In the proposed concept, a surgical or scientific imaging device is designed in such a way that either (part of) the digital imaging path can be removed and replaced with a visual imaging path including oculars, or optical elements (i.e., optical components) can be introduced into the imaging path which redirect light to a visual imaging system. In case of a system failure, the digital path can now be replaced with a visual one, allowing the surgery to continue. While it is necessary to perform some manual actions to complete this conversion, the respective procedure can still proceed as would be the case in a hybrid microscope, thereby improving the operation of the surgical or scientific imaging device in case of unavailability of the digital imaging pipeline due to loss of power.

Some aspects of the present disclosure relate to a surgical or scientific imaging device. The surgical or scientific imaging device comprises a plurality of optical components arranged within an optical path of the surgical or scientific imaging device. The surgical or scientific imaging device comprises at least one optical imaging sensor configured to generate image data based on light transmitted via the optical path during a digital operation of the surgical or scientific imaging device. The surgical or scientific imaging device comprises an interface for attaching an auxiliary optical system to the surgical or scientific imaging device in case of unavailability of the digital operation. The interface provides access to the surgical or scientific imaging device for inserting an optical component of the auxiliary optical system into the optical path of the surgical or scientific imaging device. The optical component of the auxiliary optical system is suitable for diverting the light transmitted via the optical path to the auxiliary optical system. This way, the light can be diverted into the auxiliary optical system, where it can be directed towards oculars that can be used by the scientist or surgeon to continue the respective operation. While it is necessary to perform some manual actions to attach the auxiliary optical system, the respective procedure can still continue as would be the case in a hybrid or fully visual microscope, thereby ensuring the operation of the surgical or scientific imaging device in case of unavailability of the digital imaging pipeline due to loss of power. Moreover, the construction of the surgical or scientific imaging device may remain compact, as the fully visual data path is only attached when it is needed in case of unavailability of the digital imaging pipeline.

As outlined above, at least two approaches may be used for diverting the light into the auxiliary optical system - in a first approach, an optical component of the surgical or scientific imaging device may be replaced, while, in a second approach, if there is sufficient space between the existing optical components of the surgical or scientific imaging device, an optical component may be added to the existing optical components. In other words, in the first approach, the plurality of optical components may comprise at least one optical component that is removable to allow for insertion of the at least one optical component of the auxiliary optical system into the optical path of the surgical or scientific imaging device. This way, light can be diverted without requiring an increase in the size of the surgical or scientific imaging device to accommodate space for inserting the additional optical component of the auxiliary optical system.

To allow for easy removal of the removable optical component and subsequent easy attachment of the auxiliary optical system, a modular approach may be used, in which the removable optical component is hosted in a removable sub-housing. In other words, the surgical or scientific imaging device may comprise a housing and the at least one optical component being removable may be arranged in a removable sub-housing that can be removed from the housing to allow for insertion of the at least one optical component. This way, removal of the removable optical component is facilitated, particularly in scenarios where there is only limited light due to power loss.

As a result of the modular approach, the interface for attaching the auxiliary optical system to the surgical or scientific imaging device may be the same interface that is used to attach the removable sub-housing to the surgical or scientific imaging device. In other words, the interface for attaching the auxiliary optical system to the surgical or scientific imaging device may be further configured to attach the sub-housing to the surgical or scientific imaging device. This way, the interface can be reused, resulting in fewer components needed for the construction of the surgical or scientific imaging device.

In compact surgical or scientific imaging devices, a prism is used to redirect light, e.g., to achieve a compact height of the surgical or scientific imaging device. To achieve this compactness, the housing of the surgical or scientific imaging device, which encloses the optical path, often follows the redirection of the light. If the prism redirects the light by 90 degrees, an opportunity is provided to replace a portion of the surgical or scientific imaging device that is arranged at a corner of the surgical or scientific imaging device, facilitating the removal and replacement of this portion of the surgical or scientific imaging device. Accordingly, the at least one optical component that is removable may comprise a prism (or another type of optical component suitable for redirecting light, e.g., by 90 degrees, such as a fully reflective mirror).

In the second approach, the optical component of the auxiliary optical system is inserted into the optical path without requiring removal of an optical component of the surgical or scientific imaging device. For example, the interface may comprise a slot for inserting the optical component of the auxiliary optical system into the optical path of the surgical or scientific imaging device without requiring removal of an optical component of the plurality of optical components. A slot may provide suitable lateral and vertical alignment of the optical component of the auxiliary optical system. Moreover, slots can be made compact, such that the construction of the overall surgical or scientific imaging device can still remain relatively compact.

To further support the compact construction of the surgical or scientific imaging device, optical components of the surgical or scientific imaging device that are not used when the auxiliary imaging system is attached may be moved out of the way to allow for insertion of the optical component of the auxiliary optical system. For example, the plurality of optical components may comprise at least one optical component that is arranged, when the auxiliary optical system is attached to the surgical or scientific imaging device and the optical component of the auxiliary optical system is inserted into the optical path, between the optical component of the auxiliary optical system and the at least one optical imaging sensor. The at least one optical component of the plurality of optical components may be movable to allow for insertion of the at least one optical component of the auxiliary optical system into the optical path of the surgical or scientific imaging device. This way, the space required for inserting the optical component of the auxiliary optical system may be kept low.

For example, the at least one optical component being movable may be part of a zoom system of the surgical or scientific imaging device. Zoom systems include movable components, making them suitable for being moved out of the way to make space for inserting the optical component of the auxiliary optical system.

In case of loss of power or if the digital operation of the surgical or scientific imaging device is unavailable, the electronic zoom mechanism may be unavailable as well. Thus, the surgical or scientific imaging device may comprise a mechanism for manually moving the at least one optical component. This way, the at least one optical component being movable may be moved out of the way while the digital operation of the surgical or scientific imaging device is unavailable.

In general, both digital and optical microscopy rely on light to illuminate the sample. In many surgical or scientific imaging devices, this light is provided by an illumination system that is part of or coupled with the surgical or scientific imaging device and may also be unavailable in case of power loss. To provide suitable lighting for the operation being performed using the imaging device, a battery-powered light source may be used. For example, the surgical or scientific imaging device further may comprise a second interface for attaching a battery-powered light source to the surgical or scientific imaging device. This way, the battery-powered light source can be placed close to the sample being imaged without impeding the operation of the surgical or scientific imaging device. Alternatively, the battery-powered light source may be integrated in the auxiliary optical system.

In general, the weight of the auxiliary optical system, including its housing, optical components, oculars, etc., may have an impact on the balance of the surgical or scientific imaging device, which may cause the surgical or scientific imaging device to tilt during operation. Thus, the surgical or scientific imaging device further may comprise a third interface for attaching a counterweight to the surgical or scientific imaging device. This way, the balance may be kept while using the auxiliary optical system.

During operation of the auxiliary optical system, it may be considered important that mechanical stability and alignment between the auxiliary optical system and the surgical or scientific imaging device is maintained. While this can be achieved by attaching the auxiliary optical system using a number of screws, this may be impractical, in particular if the auxiliary optical system is to be attached under adverse lighting conditions. Thus, a locking mechanism or locking structure may be used that can be used to fasten the auxiliary optical system to the surgical or scientific imaging device without requiring screws, bolts etc. For example, the interface may comprise a bayonet mount or a locking mechanism (such as a latch). This way, mechanical stability and alignment between the auxiliary optical system and the surgical or scientific imaging device can be provided without requiring a time-consuming mounting procedure.

Another aspect of the present disclosure relates to a corresponding auxiliary optical system for a surgical or scientific imaging device. The auxiliary optical system comprises an interface for attaching the auxiliary optical system to the surgical or scientific imaging device. The auxiliary optical system comprises a plurality of optical components. The plurality of optical components comprises an optical component to be inserted into an optical path of the surgical or scientific imaging device. The optical component is configured to divert light transmitted via the optical path of the surgical or scientific imaging device to the auxiliary optical system. The auxiliary optical system comprises one or more oculars for providing a view on a sample through the plurality of optical components and at least a portion of the optical path of the surgical or scientific imaging device. By attaching the auxiliary optical system to the surgical or scientific imaging device, the respective procedure can still continue as it would in a hybrid microscope, thereby improving the operation of the surgical or scientific imaging device in case of unavailability of the digital imaging pipeline due to loss of power.

As outlined above, in a first approach, an optical component of the surgical or scientific imaging device may be replaced. Accordingly, the optical component of the auxiliary imaging system to be inserted into an optical path may be configured to replace at least one optical component of the surgical or scientific imaging device when the auxiliary optical system is attached to the surgical or scientific imaging device. This way, the light can be diverted without requiring the size of the surgical or scientific imaging device to be increased to account for space to insert the additional optical component of the auxiliary optical system.

To allow for easy removal of the removable optical component and subsequent easy attachment of the auxiliary optical system, a modular approach may be used, in which the removable optical component of the surgical or scientific imaging device is housed in a removable sub-housing. Accordingly, the auxiliary optical system may comprise a housing that is configured to be inserted into the surgical or scientific imaging device in place of a sub-housing of the surgical or scientific imaging device. This way, removal of the removable optical component is facilitated, particularly in scenarios where there is only limited light due to power loss.

For example, the optical component to be inserted may be a prism (or other optical component) that is configured to replace a prism of the surgical or scientific imaging device. By replacing a prism of the surgical or scientific imaging device, light can be redirected towards the auxiliary optical system instead of towards the latter components of the optical path of the surgical or scientific imaging device. Moreover, as such a prism may be arranged at a corner of a housing of the surgical or scientific imaging device, removal of the prism may be facilitated.

In the second approach, the optical component of the auxiliary optical system is inserted into the optical path without requiring removal of an optical component of the surgical or scientific imaging device. Accordingly, the auxiliary optical system may comprise an insert to be inserted into a slot of the surgical or scientific imaging device. For example, the insert may comprise the optical component configured to divert the light to the auxiliary optical system. By providing an insert that is to be inserted into a slot, mechanical stability of the attachment can be achieved at a low footprint at the surgical or scientific imaging device.

In general, both digital and optical microscopy rely on light to illuminate the sample. In many surgical or scientific imaging devices, this light is provided by an illumination system that is part of or coupled with the surgical or scientific imaging device and which may also be unavailable in case of power loss. To provide suitable lighting for the operation being performed using the imaging device, a battery-powered light source may be used. For example, the auxiliary optical system may comprise at least one light source for illuminating the sample through at least the portion of the optical path of the surgical or scientific imaging device. For example, the auxiliary optical system further may comprise a battery for powering the at least one light source, and/or a connector for connecting the at least one light source to an external power source. This way, illumination of the sample can be provided even in case of power loss or unavailability of the illumination system of the surgical or scientific imaging device. Including a battery facilitates attachment of the auxiliary optical system to the surgical or scientific imaging device, as there is no need for routing an additional power cable somewhere along the surgical or scientific imaging device. Using an external battery means that the battery can be replaced with little effort, e.g., in case the loss of power takes a long time to recover.

Another aspect of the present disclosure relates to a system comprising the surgical or scientific imaging device and the auxiliary optical system.

For example, the system may further comprise a battery-powered light source that may be independent from the auxiliary optical system and/or the surgical or scientific imaging system (i.e., not included in either of these optical systems). This way, illumination of the sample can be provided even in case of power loss or unavailability of the illumination system of the surgical or scientific imaging device.

In another example, the system may further comprise a counterweight. This way, the balance can be maintained while using the auxiliary optical system.

### Short description of the Figures

Some examples of apparatuses and/or methods will be described in the following by way of example only, and with reference to the accompanying figures, in which:
- Fig. 1a: shows a schematic diagram of a surgical imaging system;
- Fig. 1b: shows a schematic diagram of optical components of a surgical or scientific imaging device;
- Figs. 2a and 2b: show schematic diagrams of another example of optical components of a surgical or scientific imaging device, illustrating how a prism of the surgical or scientific imaging device is replaced by a prism of an auxiliary optical system;
- Fig. 3: shows a schematic diagram of another example of optical components of a surgical or scientific imaging device, illustrating how a prism of an auxiliary optical system is inserted into an optical path of the surgical or scientific imaging device;
- Fig. 4: shows a schematic diagram of another example of optical components of a surgical or scientific imaging device, illustrating how a battery-powered light source can be mounted at an objective of the surgical or scientific imaging device; and
- Fig. 5: shows a schematic diagram of another example of optical components of a surgical or scientific imaging device, illustrating how a counterweight can be used to maintain stability of the surgical or scientific imaging device when an auxiliary optical system is attached.

### Detailed Description

Various examples will now be described more fully with reference to the accompanying drawings in which some examples are illustrated. In the figures, the thicknesses of lines, layers and/or regions may be exaggerated for clarity.

The present disclosure relates to surgical and scientific imaging devices. Surgical or scientific imaging devices are specialized tools used to capture detailed images or videos within scientific or medical contexts, allowing professionals to examine small or internal structures that are often not visible to the naked eye. Examples of such devices include microscopes, which magnify tiny samples for research or diagnostic purposes; exoscopes, which provide high-resolution images of surgical fields; and endoscopes, which enable visualization of the inside of the body through small incisions or natural openings. These devices differ from other types of imaging devices, such as digital cameras, in that they are specifically designed for high precision and clarity in detailed, often confined environments, rather than for general imaging.

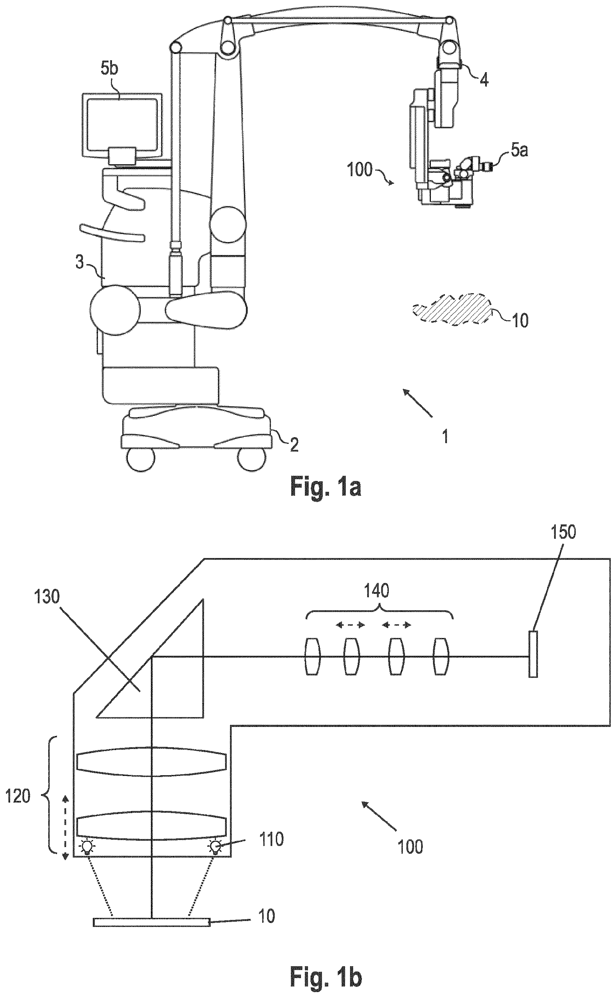

Surgical or scientific imaging devices are often used as part of a larger system, which may include additional components that are used in combination with the respective surgical or scientific imaging device. For example, Fig. 1a shows a schematic diagram of a surgical imaging system 1 comprising a surgical imaging device 100. For example, the surgical imaging system 1 may comprise one or more additional components, such as one or more of a rolling base 2, a control system 3 for controlling various functionalities of the surgical imaging system 1 and performing image processing for the surgical imaging device 100, a robotic arm 4 attached to the base 2 and the surgical imaging device 100, and ocular displays 5a and/or an auxiliary display 5b. In the following, the surgical imaging system 1 may also be referred to as a surgical microscope system 1. A surgical microscope system is a surgical imaging system 1 that comprises a (surgical) microscope as the imaging device 100.

However, the proposed concept is not limited to such embodiments. The surgical imaging system 1 may be based on various (single or multiple) imaging devices, such as one or more microscopes, and/or one or more exoscopes (also sometimes called an extracorporeal telescope). In general, a microscope, such as the microscope 100, is an optical instrument that is suitable for examining objects that are too small to be examined by the human eye (alone). For example, a microscope may provide optical magnification of a sample. In the present concept, the optical magnification is provided for at least one optical imaging sensor. Exoscopes are camera-based imaging systems, and, in particular, camera-based 3D imaging systems, which are suitable for providing images of surgical sites with high magnification and a large depth of field. Compared to microscopes, which may be used via oculars, exoscopes are only used via display modalities, such as a monitor or a head-mounted display. Accordingly, the surgical imaging system 1 may alternatively be a surgical endoscope system, or a surgical exoscope system. The following illustrations assume that the surgical or scientific imaging device 1 is a exoscope, and that the surgical imaging system 1 is a surgical exoscope system 1. For example, in a surgical imaging system (as well as a scientific imaging system), the surgical imaging device is also denoted 'optics carrier'.

However, the proposed concept is not limited to surgical imaging devices and surgical imaging systems. For example, the imaging device 100 may be a laboratory microscope. A laboratory microscope is designed for examining slides of specimens at high magnification for research and diagnostic purposes, often featuring multiple objective lenses for different magnification levels.

Conventional hybrid microscopes split the light between a digital and a visual imaging path to provide a visual image through the oculars and a digital image on a screen. The advantage of this is that an image will always be available even during power supply issues or other system failures, although the quality of the image might be impacted due to lack of illumination. Pursuing a hybrid approach may result in a larger optics carrier and reduced image quality/higher required illumination power as the available light is split into two paths. A fully digital imaging device (such as the exoscope shown in Fig. 1b) uses all available light on the only digital beam path, thus increasing the image quality. In a fully digital system, a system failure may result in loss of any image.

In the proposed concept, the digital surgical or scientific imaging device, e.g., the exoscope, is designed in a way that allows either (part of) the digital imaging path to be removed and replaced with a visual imaging path including oculars or optical elements (i.e., components) to be introduced into the imaging path redirecting the light to the visual imaging system. In case of a system failure, the digital path can now be replaced with a visual one, allowing the surgery to continue or safely ended. The main difference from traditional hybrid microscope designs is that traditionally the light is split between the different light paths and the required optical elements are always in place. In the proposed concept, the optical components are replaced or inserted only when needed, ensuring 100% of the light is available in the used beam path. Further, a small footprint is maintained during normal operation, as the unused components are removed, allowing full view of the monitor.

The visual system (also denoted auxiliary visual system in the following) can be designed in a straightforward manner, only providing fixed magnification (no zoom lenses) and no fluorescence imaging (no filters) to reduce size, weight, and cost. Battery-powered illumination can be included in the replacement system to provide a light source in case of power failure. The replacement system (auxiliary visual system) may be stored in the exoscope/microscope stand 2, attached to a charger to always keep the batteries charged for emergencies.

Fig. 1b shows a schematic diagram of optical components of a digital surgical or scientific imaging device 100. The digital surgical or scientific imaging device 100 comprises light sources 110 that are arranged at the bottom of the housing towards the sample 10 to be imaged. A multifocus system 120 is provided to allow the operator to capture images at multiple focal depths (e.g., simultaneously or in rapid succession). A prism 130 is used to redirect the light by 90 degrees towards a zoom system 140 and an optical imaging sensor 150 (e.g., a camera). The multifocus system 120, the prism 130 and the zoom system 140 are optical components of the digital surgical or scientific imaging device 100. The optical path of the digital surgical or scientific imaging device 100 traverses the multifocus system 120, the prism 130, the zoom system 140, ending at the optical imaging sensor 150 and, outside the digital surgical or scientific imaging device 100, at the sample 10.

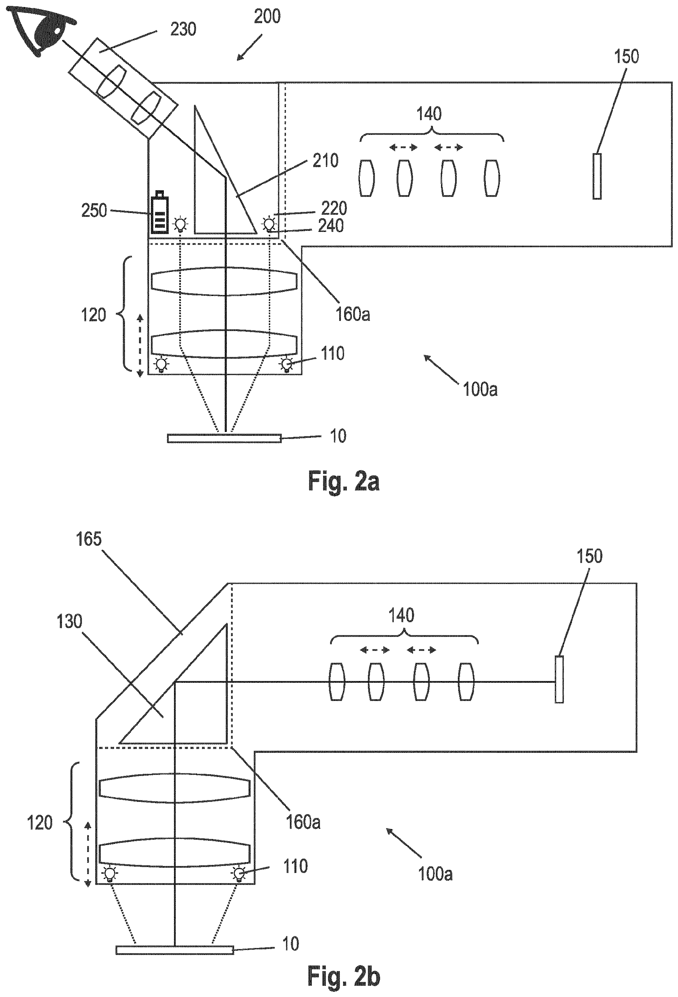

In connection with Figs. 2a to 5, an improved digital surgical or scientific imaging device 100a, 100b, 100c, 100d is provided, which allows for the attachment of an auxiliary optical system 200, 300, 400, 500 to provide an optical path between oculars 230, 330, 430, 530 of the auxiliary optical system and the sample while reusing a portion of the optical path of the respective improved digital surgical or scientific imaging device 100a, 100b, 100c, 100d.

Figs. 2a to 5 show schematic diagrams of examples of a surgical or scientific imaging device 100a, 100b, 100c, 100d. The surgical or scientific imaging device 100a, 100b, 100c, 100d comprises a plurality of optical components (e.g., multifocus system 120, prism 130, zoom system 140) arranged within an optical path of the surgical or scientific imaging device. The surgical or scientific imaging device 100a, 100b, 100c, 100d comprises at least one optical imaging sensor 150 configured to generate image data based on light transmitted via the optical path during a digital operation of the surgical or scientific imaging device. The surgical or scientific imaging device 100a, 100b, 100c, 100d comprises a (mechanical) interface 160a, 160b, 160c, 160d for attaching an auxiliary optical system 200, 300, 400, 500 to the surgical or scientific imaging device in case of unavailability of the digital operation. The interface provides access to the surgical or scientific imaging device for inserting an optical component 210, 315, 415, 515 of the auxiliary optical system into the optical path of the surgical or scientific imaging device for diverting the light transmitted via the optical path to the auxiliary optical system 200, 300, 400, 500.

Figs. 2a and 3 to 5 further show schematic diagrams of examples of an auxiliary optical system 200, 300, 400, 500 for a surgical or scientific imaging device 100a, 100b, 100c, 100d. The auxiliary optical system 200, 300, 400, 500 comprises an interface 220, 310, 410, 510 for attaching the auxiliary optical system to the surgical or scientific imaging device. The auxiliary optical system 200, 300, 400, 500 comprises a plurality of optical components 210, 315, 325, 415, 425, 515, 525. The plurality of optical components comprises an optical component 210, 315, 415, 616 to be inserted into an optical path of the surgical or scientific imaging device. The optical component is configured to divert light transmitted via the optical path of the surgical or scientific imaging device to the auxiliary optical system. The plurality of optical components comprises one or more oculars 230, 330, 430, 530 for providing a view on a sample through the plurality of optical components and at least a portion of the optical path of the surgical or scientific imaging device.

Figs. 2a and 3 to 5 further show schematic diagrams of examples of a system comprising the surgical or scientific imaging device 100a, 100b, 100c, 100d and the auxiliary optical system 200, 300, 400, 500.

More details and aspects of the surgical or scientific imaging device 100 and the surgical or scientific imaging system 1 are mentioned in connection with the proposed concept or one or more examples described above or below (e.g., Figs. 2a to 5). The surgical or scientific imaging device 100 and the surgical or scientific imaging system 1 may comprise one or more additional optional features corresponding to one or more aspects of the proposed concept or one or more examples described above or below.

Figs. 2a to 5 show various options for implementing such a surgical or scientific imaging device 100a, 100b, 100c, 100d and auxiliary optical system 200, 300, 400, 500.

In the example shown in Figs. 2a and 2b, the prism 130 changing the direction of the optical path is removed and replaced by another prism 210 directing the image to oculars 230 providing a visual image to the user. Thus, in the example shown in Figs. 2a and 2b, the prism 130 is a removable component can be removed to allow for insertion of an optical component 210 (another prism 210) of the auxiliary optical system 200 into the optical path of the surgical or scientific imaging device. When the auxiliary optical system 200 is attached to the surgical or scientific imaging device 100a (and prism 130 is removed) via the interfaces 160a, 220, the optical component (prism 210) of the auxiliary optical system 200 replaces the optical component (prism 130) of the surgical or scientific imaging device 100a. As shown in Fig. 2a, instead of redirecting the light towards the zoom system 140, prism 210 of the auxiliary optical system 200 redirects the light towards the oculars 230 of the auxiliary optical system 200.

To facilitate replacing the optical component 130 of the surgical or scientific imaging device 100a with the optical component 210 of the auxiliary optical system 200, the optical component (prism 130) of the surgical or scientific imaging device 100a may be placed into a removable sub-housing 165 (shown in Fig. 2b). The removable optical component (prism 130) may be arranged in a removable sub-housing 165 that can be removed from a housing of the surgical or scientific imaging device 100a to allow for insertion/attachment of the at least one optical component of the auxiliary optical system 200. In place of the removable sub-housing, the housing 220 of the auxiliary optical system 200 may be inserted/attached to the surgical or scientific imaging device 100a.

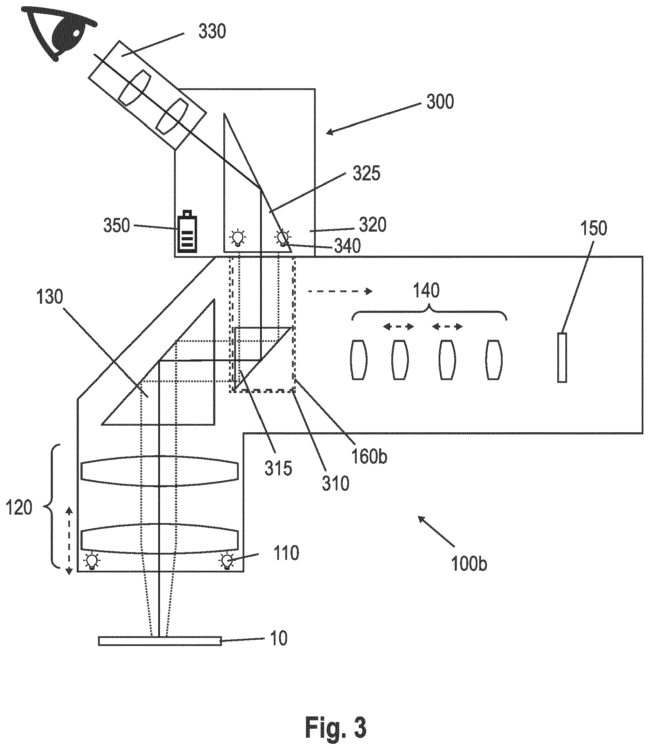

In the configuration shown in Figs. 2a and 2b, the interface 160a is used both to attach the sub-housing 165 to the housing of the surgical or scientific imaging device 100a and to attach the housing 220 of the auxiliary optical system 200 to the surgical or scientific imaging device 100a in place of the removable sub-housing. More generally, and also with reference to Figs. 3 to 5, the interface 160 and corresponding interface 220, 310, 410, 510 of the auxiliary optical system 200, 300, 400, 500 are used to mechanically couple the respective auxiliary optical system 200, 300, 400, 500 to the surgical or scientific imaging device 100a, 100b, 100c, 100d. The respective interfaces 160, 220, 310, 410, 510 provide a reversible mechanical coupling of the auxiliary optical system 200, 300, 400, 500 to the surgical or scientific imaging device 100a, 100b, 100c, 100d as well as an optical alignment between the optical components (e.g., multifocus system 120, prism 130 in case of Figs. 3 to 5) of the surgical or scientific imaging device 100a, 100b, 100c, 100d and the optical components (prisms 210, 315, 325, 415, 425, 515, 525 and oculars 230, 330, 430, 450) of the auxiliary optical system 200, 300, 400, 500. The interfaces 160, 220, 310, 410, 510 are provided to provide an easy and secure attachment of the auxiliary optical system 200, 300, 400, 500 to the surgical or scientific imaging device 100a, 100b, 100c, 100d. For this purpose, the interface 160 of the surgical or scientific imaging device 100a, 100b, 100c, 100d or the interface 220, 310, 410, 510 of the auxiliary optical system 200, 300, 400, 500 may comprise a reversible (in the sense of enabling reversing the attachment to the imaging device) locking mechanism, such as a bayonet mount or latching mechanism (e.g., a latch or catch of the latching mechanism, a spring and an actuator), with the respective other interface comprising a corresponding bayonet fitting or strike plate (that the latch or catch engages with).

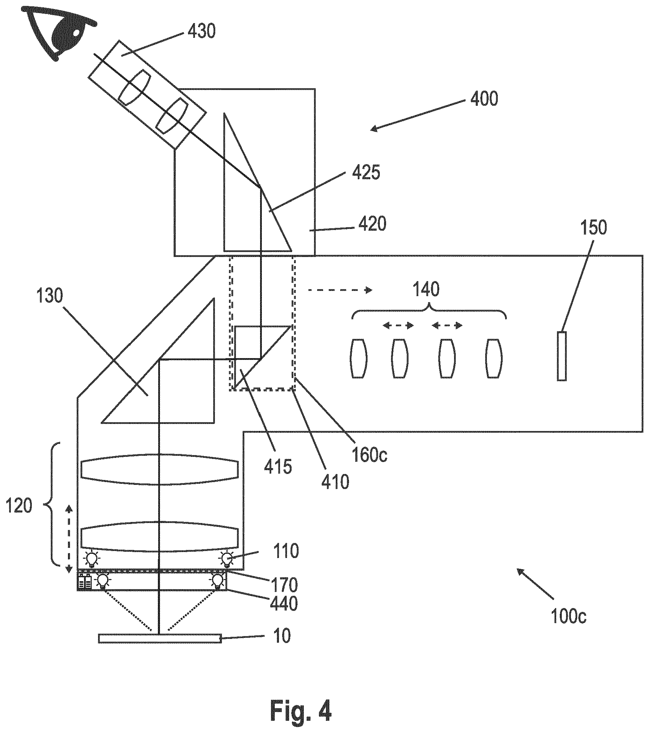

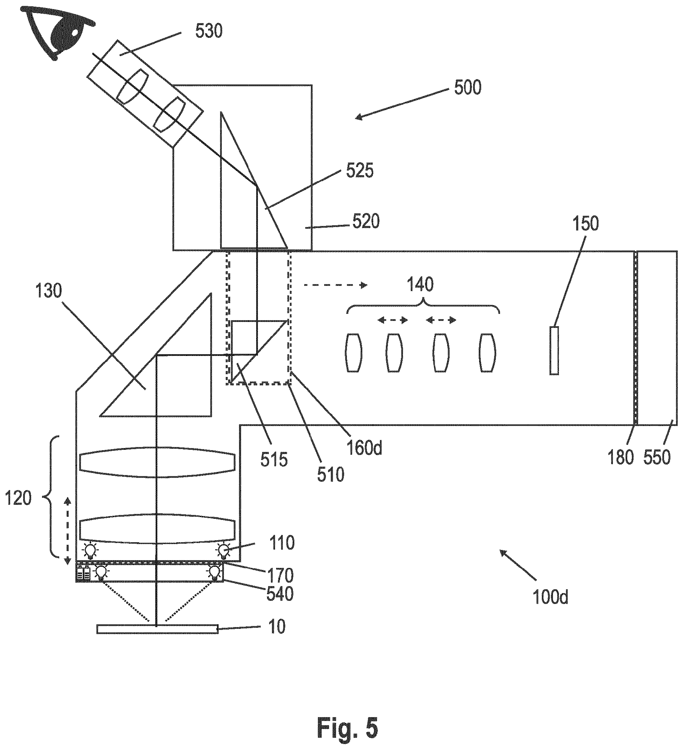

In Figs. 2a and 2b, the auxiliary optical system 200 optionally further comprises at least one light source 240 for illuminating the sample through at least the portion of the optical path of the surgical or scientific imaging device. In other words, the light follows the optical path through some of the optical components 210, 120 of the auxiliary optical system 200 and of the surgical or scientific imaging device 100a. Further optionally, the auxiliary optical system may comprise a battery 250 for powering the at least one light source or a connector for connecting the at least one light source to an external power source. However, the replacement-based approach shown in Figs. 2a and 2b is not limited to including the light source 240. For example, as an alternative, a (ring-shaped) light source may be placed around the objective of the exoscope/microscope, e.g., as shown in Figs. 4 and 5. Further alternatively, illumination may be provided by an external illumination system.

The approach shown in Figs. 2a and 2b is simple in terms of optical components. As the main prism is removed and placed again to restore normal system operation, if the prism is not placed accurately, this might result in poor alignment of the optical path and cameras.

More details and aspects of the surgical or scientific imaging device 100a and the auxiliary optical system 200 are mentioned in connection with the proposed concept or one or more examples described above or below (e.g., Figs. 1a to 1b and 3 to 4). The surgical or scientific imaging device 100a and the auxiliary optical system 200 may comprise one or more additional optional features corresponding to one or more aspects of the proposed concept or one or more examples described above or below.

In Fig. 3, a different configuration is shown. Fig. 3 shows a schematic diagram of another example of optical components of a surgical or scientific imaging device 100b, illustrating how a prism 315 of an auxiliary optical system 300 is inserted into the optical path of the surgical or scientific imaging device 100c.

In the examples shown in Figs. 3 to 5, instead of replacing the main prism 130 of the surgical or scientific imaging device, the optical component 315, 415, 515 of the auxiliary optical system 300, 400, 500 is inserted into the optical path without removing any of the optical components of the respective surgical or scientific imaging device 100b, 100c, 100d. Instead, a prism 315, 415, 515 (or mirror) is inserted into the optical path between the main prism 130 and the zoom system 140. For example, the additional prism 315, 415, 515 may be placed in the optical path in front of the zoom system 140 to direct the image to the oculars 330, 430, 450.

In particular, a slot 160b, 160c, 160d may be provided as interface for inserting the optical component 315, 415, 515 of the auxiliary optical system into the optical path of the surgical or scientific imaging device without requiring removal of an optical component of the respective surgical or scientific imaging device 100b, 100c, 100d. In this context, the (mechanical) slot is a narrow opening in the housing of the surgical or scientific imaging device 100b, 100c, 100d, designed to receive or hold specific components, such as an insert 310, 410, 510 comprising the optical component 315, 415, 515 to be inserted into the optical path. The insert can be inserted into a slot by aligning it with the opening and applying gentle pressure until it slides into place, e.g., guided by rails or channels along the sides of the slot. To lock the insert into a slot, the aforementioned locking mechanism (latch/snap-fit) can be employed that creates a mechanical interference when the insert reaches its fully seated position. When not in use, the slot can be covered with a protective cap, sliding door, hinged flap, or dedicated blank insert that fits the slot dimensions, preventing dust accumulation.

In general, compactness is one major benefit of fully digital surgical or scientific imaging devices. This means that there usually is no spare room for inserting the optical component of the auxiliary optical system 300, 400, 500. To make room for the insert, at least one of the zoom lenses of the zoom system 140 may be moved out of the way to make space for the insert. More generally, the optical components may comprise at least one optical component 140 (e.g., at least one lens of the zoom system 140) that is arranged, when the auxiliary optical system is attached to the surgical or scientific imaging device and the optical component of the auxiliary optical system is inserted into the optical path, between the optical component 315, 415, 515 of the auxiliary optical system and the at least one optical imaging sensor 150. As the light is redirected towards the auxiliary optical system before it reaches this optical component, it is of no use during attachment of the auxiliary optical system 300, 400, 500. Therefore, it may be moved out of the way to make space for the optical component 315, 415, 515 of the auxiliary optical system. In other words, the at least one optical component being arranged between the optical imaging sensor 150 and the optical component 315, 415, 515 of the auxiliary optical system, may be movable to allow for insertion of the at least one optical component of the auxiliary optical system into the optical path of the surgical or scientific imaging device. For example, the zoom lens(es) may be manually moved to the end of the zoom range to provide space to insert the additional prism. Accordingly, the surgical or scientific imaging device may comprise a mechanism, such as a mechanical zoom ring or a lever, for manually moving the at least one optical component (e.g., the zoom lens).

Similar to the configuration shown in Figs. 2a and 2b, the auxiliary optical system 300 optionally further comprises at least one light source 340 for illuminating the sample through at least a portion of the optical path of the surgical or scientific imaging device. Furthermore, the auxiliary optical system may optionally comprise a battery 350 for powering the at least one light source or a connector for connecting the at least one light source to an external power source. For example, as an alternative, a (ring-shaped) light source may be placed around the objective of the exoscope/microscope, e.g., as shown in Figs. 4 and 5. Alternatively, illumination may be provided by an external illumination system.

In the approach shown in Figs. 3 to 5, additional optical components are required, but none of the components in the digital imaging path need to be removed, thus reducing the risk of misalignment of the optics. This system may be considered similar to a conventional hybrid microscope, except that the conventional hybrid microscope includes a permanent beamsplitter that directs part of the light to the camera and part to the visual path. In the approach shown in Figs. 3 to 5, the beamsplitter is replaced by a removable prism so that always 100% of the light is available in the mode being used, providing better image quality and/or reducing the amount of required illumination.

More details and aspects of the surgical or scientific imaging device 100b and the auxiliary optical system 300 are mentioned in connection with the proposed concept or one or more examples described above or below (e.g., Figs. 1a to 2b, 4 to 5). The surgical or scientific imaging device 100b and the auxiliary optical system 300 may comprise one or more additional optional features corresponding to one or more aspects of the proposed concept or one or more examples described above or below.

A possible drawback of the configuration shown in Figs. 2a and 3 is that the illumination is directed through the imaging optics, which can cause back reflections on lens surfaces and result in glare in the image. In general, the configurations shown in Figs. 4 and 5 are similar to the configuration shown in Fig. 3. However, in the configuration shown in Figs. 4 and 5, the illumination is moved out of the replacement imaging system (i.e., the auxiliary optical system). A second attachable part 440, 540 is provided that only includes the illumination. This extra part 440, 550, which may be ring-shaped, may be attached to the objective and thus avoid the back reflection issue, at the cost of increasing the complexity of the surgical or scientific imaging device.

Fig. 4 shows a schematic diagram of another example of optical components of a surgical or scientific imaging device, illustrating how a battery-powered light source 440 can be mounted at an objective of the surgical or scientific imaging device 100c, 100d. To host the battery-powered light source 440, the surgical or scientific imaging device 100c, 100d may further comprise a second (mechanical) interface 170 for attaching a battery-powered light source 440, 540 to the surgical or scientific imaging device. For example, the second interface 170 may be arranged at the objective of the surgical or scientific imaging device 100c, 100d. It may be used to attach the battery-powered light source to the surgical or scientific imaging device 100c, 100d. Again, the interface 170 may comprise a bayonet mount or locking mechanism (e.g., latching mechanism or snap-fit mechanism) for removably attaching the battery-powered light source to the surgical or scientific imaging device 100c, 100d. For example, the surgical or scientific imaging device 100c, 100d may comprise the battery or batteries for powering the battery-powered light source, or a connector for connecting an external battery to the battery-powered light source.

While the use of a separate battery-powered light source is discussed in connection with the insertion-based auxiliary optical system of Figs. 3, 4 and 5, it may likewise be applied to the replacement-based auxiliary optical system of Figs. 2a and 2b.

More details and aspects of the surgical or scientific imaging device 100c and the auxiliary optical system 400 are mentioned in connection with the proposed concept or one or more examples described above or below (e.g., Figs. 1a to 3, 5). The surgical or scientific imaging device 100c and the auxiliary optical system 400 may comprise one or more additional optional features corresponding to one or more aspects of the proposed concept or one or more examples described above or below.

In some cases, the addition of new components, such as the oculars, may unbalance the optics carrier. Therefore, a counterweight 550 may be added on the other side of the optics carrier to restore balance and ensure ease of movement. Fig. 5 shows a schematic diagram of another example of optical components of a surgical or scientific imaging device 100d, illustrating how a counterweight 550 can be used to maintain stability of the surgical or scientific imaging device when an auxiliary optical system 500 is attached. For example, the counterweight may be added at a side of the surgical or scientific imaging device 100d that is opposite the side where the oculars 530 of the auxiliary optical system 500 are facing. As the counterweight might unbalance the surgical or scientific imaging device 100d when the auxiliary optical system 500 is not attached, it may be attached via a third interface 180 of the surgical or scientific imaging device 100d. In other words, the surgical or scientific imaging device 100d may further comprise a third interface 180 for attaching the counterweight 550 to the surgical or scientific imaging device 100d. For example, the third interface may be arranged at a side of the surgical or scientific imaging device 100d that is opposite the side where the oculars 530 of the auxiliary optical system 500 are facing. It is a mechanical interface for holding the counterweight in place. For example, the third interface 180 may comprise a bayonet mount or locking mechanism (latching mechanism/snap-fit mechanism) for removably holding the counterweight in place. The use of a counterweight may improve the balance of the surgical or scientific imaging system, albeit at an increased complexity.

While the use of a separate battery-powered light source is discussed in connection with the insertion-based auxiliary optical system of Fig. 5, it may likewise be applied to the replacement-based auxiliary optical system of Figs. 2a and 2b.

More details and aspects of the surgical or scientific imaging device 100d and the auxiliary optical system 500 are mentioned in connection with the proposed concept or one or more examples described above or below (e.g., Figs. 1a to 4). The surgical or scientific imaging device 100d and the auxiliary optical system 500 may comprise one or more additional optional features corresponding to one or more aspects of the proposed concept or one or more examples described above or below.

In general, surgical or scientific imaging devices may be used with a drape, with the drape providing a sterile covering that protects the surgical or scientific imaging device from contamination while maintaining a sterile field, e.g., during surgery. The auxiliary optical system 300, 400, 500, 600 may be considered in the design of the drape. For example, the light source component 440, 540 may be directly connected to the drape lens adapter. For example, the counterweight may be attached above the drape. Only the oculars might be provided with an opening in the drape, which can be designed similarly to the ocular openings in current microscopes.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference Signs

1 Surgical imaging system
2 Rollable stand
3 Control system
4 Robotic arm
5a Ocular displays
5b Auxiliary display
10 Sample
100, 100a, 100b, 100c, 100d Surgical or scientific imaging device
110 Light source
120 Multifocus system
130 Main prism
140 Zoom system
150 Optical imaging sensor
160, 160a, 160b, 160c, 160d Interface for auxiliary optical system
170 Second interface for battery-powered light source
180 Third interface for counterweight
200, 300, 400, 500 Auxiliary optical system
210, 315, 415, 425 Prism inserted into optical path of surgical or scientific imaging device
220 Housing
230, 330, 430, 530 Oculars
240, 340 Light source
250, 350 Battery
325, 425, 525 Prism
440, 540 Battery-powered light source
550 Counterweight

## Claims

1. A surgical or scientific imaging device (100a, 100b, 100c, 100d), comprising:
a plurality of optical components (120, 130, 140) arranged within an optical path of the surgical or scientific imaging device;
at least one optical imaging sensor (150) configured to generate image data based on light transmitted via the optical path during a digital operation of the surgical or scientific imaging device; and
an interface (160a, 160b, 160c, 160d) for attaching an auxiliary optical system (200, 300, 400, 500) to the surgical or scientific imaging device in case of unavailability of the digital operation, wherein the interface provides an access to the surgical or scientific imaging device for inserting an optical component (210, 315, 415, 515) of the auxiliary optical system into the optical path of the surgical or scientific imaging device for diverting the light transmitted via the optical path to the auxiliary optical system.

2. The surgical or scientific imaging device according to claim 1, wherein the plurality of optical components comprises at least one optical component (130) that is removable to allow for insertion of the at least one optical component of the auxiliary optical system into the optical path of the surgical or scientific imaging device.

3. The surgical or scientific imaging device according to claim 2, wherein the surgical or scientific imaging device comprises a housing and the at least one optical component being removable is arranged in a removable sub-housing (165) that can be removed from the housing to allow for insertion of the at least one optical component.

4. The surgical or scientific imaging device according to claim 3, wherein the interface (160a) for attaching the auxiliary optical system to the surgical or scientific imaging device is further configured to attach the sub-housing to the surgical or scientific imaging device.

5. The surgical or scientific imaging device according to one of the claims 2 to 4, wherein the at least one optical component that is removable comprises a prism (130).

6. The surgical or scientific imaging device according to claim 1, wherein the interface (160b, 160c, 160d) comprises a slot for inserting the optical component (315, 415, 515) of the auxiliary optical system into the optical path of the surgical or scientific imaging device without requiring removal of an optical component of the plurality of optical components.

7. The surgical or scientific imaging device according to claim 6, wherein the plurality of optical components comprises at least one optical component (140) that is arranged, when the auxiliary optical system is attached to the surgical or scientific imaging device and the optical component of the auxiliary optical system is inserted into the optical path, between the optical component (315, 415, 515) of the auxiliary optical system and the at least one optical imaging sensor (150), with the at least one optical component being movable to allow for insertion of the at least one optical component of the auxiliary optical system into the optical path of the surgical or scientific imaging device.

8. The surgical or scientific imaging device according to claim 7, wherein the at least one optical component being movable is part of a zoom system (140) of the surgical or scientific imaging device,
and/or wherein the surgical or scientific imaging device comprises a mechanism for manually moving the at least one optical component.

9. The surgical or scientific imaging device according to one of the claims 1 to 8, wherein the surgical or scientific imaging device (100c, 100d) further comprises a second interface (170) for attaching a battery-powered light source (440, 540) to the surgical or scientific imaging device,
and/or wherein the surgical or scientific imaging device (100d) further comprises a third interface (180) for attaching a counterweight (550) to the surgical or scientific imaging device.

10. The surgical or scientific imaging device according to one of the claims 1 to 9, wherein the interface (160a, 160b, 160c, 160d) comprises a bayonet mount or a locking mechanism.

11. An auxiliary optical system (200, 300, 400, 500) for a surgical or scientific imaging device (100a, 100b, 100c, 100d), comprising:
an interface (220, 310, 410, 510) for attaching the auxiliary optical system to the surgical or scientific imaging device;
a plurality of optical components (210, 315, 325, 415, 425, 515, 525), wherein the plurality of optical components comprises an optical component (210, 315, 415, 616) to be inserted into an optical path of the surgical or scientific imaging device, with the optical component being configured to divert light transmitted via the optical path of the surgical or scientific imaging device to the auxiliary optical system; and
one or more oculars (230, 330, 430, 530) for providing a view on a sample through the plurality of optical components and at least a portion of the optical path of the surgical or scientific imaging device.

12. The auxiliary optical system according to claim 11, wherein the optical component to be inserted into an optical path is configured to replace at least one optical component (130) of the surgical or scientific imaging device when the auxiliary optical system is attached to the surgical or scientific imaging device.

13. The auxiliary optical system according to claim 11, wherein the auxiliary optical system comprises an insert (310, 410, 510) to be inserted into a slot (160b, 160c, 160d) of the surgical or scientific imaging device, the insert comprising the optical component configured to divert the light to the auxiliary optical system.

14. The auxiliary optical system according to one of the claims 11 to 13, comprising at least one light source (240, 340) for illuminating the sample through at least the portion of the optical path of the surgical or scientific imaging device.

15. A system comprising the surgical or scientific imaging device (100a, 100b, 100c, 100d) according to one of the claims 1 to 10 and the auxiliary optical system (200, 300, 400, 500) according to one of the claims 11 to 14.
